# EUROPÄISCHE PATENTANMELDUNG

(11) **EP 3 363 419 A1**
(43) Veröffentlichungstag der Anmeldung: **22.08.2018**
(21) Anmeldenummer: 18157899.8
(22) Anmeldetag: 21.02.2018
(51) Int. Cl.: A61H 9/00, A61B 17/132, A61B 17/135, A63B 23/035, A63B 21/00, A63B 21/008

(54) **PNEUMATISCHES ADERPRESS-SYSTEM**

(30) Priorität: 21.02.2017 DE 102017103504
(71) Anmelder: University of Ljubljana, 1000 Ljubljana (SI)
(72) Erfinder: KACIN, Alan, 1000 Ljubljana (SI); JELENC, Jure, 4244 Podnart (SI)
(74) Vertreter: Tappe, Udo

(57) **Zusammenfassung**

Um bei einem pneumatischen Aderpress-System für die Konditionierung von Muskel- und Blutgefäßen mit einer Manschette (1) mit mindestens zwei aufblähbaren Kammern (2, 3) und mit einem Druck-Regelsystem (8) zum definierten Aufblähen der Manschette (1) eine präzisere Reduzierung des Blutflusses in Körpergliedern bei gering anwachsendem Manschettendruck zu ermöglichen und somit weniger Unbehagen und Schmerzreiz sowohl in der Ruhephase als auch in der Bewegung auszulösen, sind die mindestens zwei aufblähbaren Kammern (2, 3) in Längsrichtung der Manschette (1) aufeinander folgend angeordnet sind und durch das Druck-Regelsystem (8) unabhängig voneinander gesteuert aufblähbar, wobei eine erste Kammer (2) zur Anwendung an den Muskeln und eine zweite Kammer (3) zur Anwendung an den Blutgefäßen (12) vorgesehen ist, wodurch eine asymmetrische Kompression des menschlichen Köperteils erzeugt werden kann.

## Beschreibung

Die Erfindung betrifft ein pneumatisches Aderpress-System für die Konditionierung von Muskel- und Blutgefäßen mit einer Manschette mit mindestens zwei aufblähbaren Kammern und mit einem Druck-Regelsystem zum definierten Aufblähen der Manschette.

Pneumatische Aderpress-Systeme werden für die Muskel- und Blutgefäß-Konditionierung eingesetzt, zur Reduzierung von arteriellem und venösem Blutfluss in ein Körperglied während jeglicher Art der Muskelkontraktion oder um das Eindringen von Arterienblut in ein Körperglied des Patienten während einer Operation zu stoppen oder um diese Prozedur zu unterstützen oder in jeglichen anderen Situationen, in denen eine Reduktion des Blutflusses verlangt wird. Ein solches Aderpress-System wird auch als Tourniquet-Systeme bezeichnet.

Pneumatische Aderpress-Systeme zur Reduzierung des Blutflusses finden sowohl bei Gesundheitspersonal als auch bei der Allgemeinbevölkerung einen bedeutenden Einsatz für die Messung von systolischem und diastolischem arteriellem Blutdruck.

Zu diesem Zweck wird die Manschette um ein oberes oder unteres Körperglied platziert und die Kammer, ein pneumatisches Kissen, mit Luft gefüllt, um das Körperglied über den individuellen systolischen Blutdruck hinaus zusammen zu pressen. Der Druck in der Manschette wird anschließend schrittweise reduziert und aufgezeichnet, bei gleichzeitigem Abhorchen des Pulsschlages in den Gefäßen distal der Manschette. Dieses Grundprinzip wird in verschiedenen technischen Ausführungsformen bei allen kommerziell auf dem Markt zur Verfügung stehenden Blutdruck-Messgeräten genutzt.

Eine ähnliche Technologie wird ebenfalls in kommerziell zur Verfügung stehenden pneumatischen Aderpress-Systemen genutzt, um den Blutfluss in ein Körperglied vollständig zu unterbinden oder um eine örtliche intravenöse Anästhesie während einer Operation zu ermöglichen (z.B. PTSii Tourniquet System, Delfi Medical Company, Kanada).

US 4,635,635 beschreibt ein chirurgisches Aderpress-System mit einer Profil-Manschette in verschiedenen Größen, welche für verschiedene kegelförmige Extremitäten genutzt werden kann und ein einzelnes pneumatisches Kissen umfasst, das durch zwei separat verbundene Kanäle aufgebläht und entleert wird; das bedeutet ein Kanal wird zum Aufblähen der Manschette verwendet und ein weiterer Kanal wird zum Entleeren der Manschette verwendet.

Einige der chirurgischen Aderpress-Systeme können Pulsschlag-Sensoren verwenden, welche distal zum Aderpress-System an dem Körperglied angebracht sind, als Biofeedback-Kontrolle für eine schnelle und automatische Anpassung des ReferenzDrucks in Realzeit, wie beschrieben in WO 2016/004519 A1.

In ähnlicher Weise beschreibt WO 2006102753 A1 die erweiterte Regulation eines Referenzdrucks in der Manschette während einer Operation, durch die Verwendung einer Dual-Port Manschette, wobei ein Anschluss für das Aufblähen und Entleeren der Manschette genutzt wird und ein zweiter Anschluss einem Druck-Kontroll-System ein sofortiges pneumatisches Feedback des erfassten Drucks zur Verfügung stellt, um eine automatische Anpassung des Manschetten-Referenzdrucks zu ermöglichen.

Wie in WO 9606568 A2 beschrieben, wird eine Manschette mit zwei pneumatischen Kissen die parallelgeschaltet entlang der Längsrichtung der Manschette angeordnet sind - das bedeutet eine Doppelmanschette - in Operationen verwendet, um die Sicherheit bei intravenösen, regionalen Anästhesien zu gewährleisten und zu erhöhen.

US 2007/0219580 A1 beschreibt ein verstellbares Design von preiswerten pneumatischen Manschetten in chirurgischen Aderpress-Systemen, welches eine optimale Anpassung der Manschette an Extremitäten verschiedener Form und Größe ermöglicht.

Die aktuellere US 2015/0190301 A1 beschreibt ein Aderpress-System für ischämische Konditionierung von Gewebe oder Organen über partielle Körperglied-Verschließung, wobei entweder eine Manschette mit einem einzelnen Kissen oder eine Doppelmanschette genutzt werden kann. Bei der beschriebenen Doppelmanschette sind die pneumatischen Kissen parallel ausgerichtet, wie in WO 9606568 A2 beschrieben. Eine andere Anordnung der entsprechenden pneumatischen Kissen ist dem Dokument nicht zu entnehmen.

EP 1 661 605 B1 und EP 1 614 451 B1 beschreiben zwei ähnliche pneumatische Geräte zur Verstärkung von Skelettmuskeln in den Körpergliedern. Beide Geräte bestehen aus schmalen bogenförmigen Manschetten mit einzelnen aufblasbaren Kissen, jedoch unterschiedlichen Verstärkungen der äußeren Manschetten-Schicht, die um das Körperglied platziert und mit einem starren Band an der äußeren Manschetten-Oberfläche via D-Ring fixiert sind.

WO 2017/ 023 951 A1 zeigt eine Vorrichtung zur Blutstillung an einer Punktionsstelle an einem Handgelenk eines Patienten. Die Vorrichtung hat ein flexibles Band, das um die Extremität eines Patienten gewickelt und mit Bindemitteln an einer Stelle an der Extremität befestigt werden kann, an der die Blutung gestoppt werden soll. Das Band hat eine Manschette mit zwei Ballons unterschiedlicher Größe. Mittels einer Spritze können die Ballons unabhängig voneinander aufgeblasen werden.

DE 197 15 461 A1 beschreibt eine Vorrichtung zur Behandlung einer Extremität des menschlichen Körpers. Ein langgestrecktes Band wird wendelförmig um und entlang der Extremität gewickelt. Das Band hat eine Anzahl an in Längsrichtung des Bandes aufeinanderfolgende Zellen. Die Zellen sind mit Einlässen versehen, um über eine Auswahleinheit mit einer Druckluftquelle verbunden zu werden. Eine progressiv wachsende Länge der Zellen von einem ersten Ende des Bandes hin zu einem zweiten Ende des Bandes kann vorgesehen sein.

Ein druckbeaufschlagtes Trainingsgerät und eine Kontroll-Methode für selbiges sind in EP 1 949 939 B1 beschrieben, wobei erwünschte Level des Luftdrucks in einer oder mehreren bogenförmigen Manschetten, die einzelne aufblasbare Kissen umfassen und die ein Körperglied umschließen, während der Muskelkontraktion unabhängig voneinander durch ein elektronisches Druck-Regelsystem reguliert werden.

EP 2 868 303 A beschreibt ein sehr ähnliches Extremitäten-Abbinde-System wie EP 1 949 939 B1, wird jedoch mit einer davon verschiedenen Druck-Kontroll-Methode (Anwendungsprotokoll) verwendet, welches auf die Blutgefäß-Konditionierung abzielt.

Verschiedene druckbeaufschlagte Trainingsgeräte, basierend auf einer Technologie beschrieben in EP 1 949 939 B1, sind kommerziell unter der Dachmarke KAATSU verfügbar (KAATSU Global, Inc., USA; Sato Sports Plaza, Ltd., Tokyo, Japan; Kaatsu Japan Co., Ltd., Tokyo, Japan).

WO 2014/117 984 A1 zeigt ein Fitnessgerät für die Konditionierung von Muskeln, mit einem Druckgürtel und mit Mitteln zum Tragen einer Pumpvorrichtung an dem Körper einer Person. Der Druckgürtel hat eine erste und eine zweite Druckkammer, wobei die Druckkammern jeweils eine Anschlusseinrichtung haben, um mittels einer Leitung mit der Pumpvorrichtung verbunden zu werden. Die Druckkammern sind dabei unabhängig voneinander steuerbar. Beide Druckkammern haben jeweils einen Basisabschnitt und Zweigabschnitte, wobei die Zweigabschnitte der beiden Druckkammern miteinander verzahnt angeordnet sind.

Pneumatische Standard Aderpress-Systeme für partielle oder vollständige Unterbindung des Blutflusses während einer Ruhephase und jeglicher Form der Muskelkontraktion in den Extremitäten, binden die Extremitäten an einer proximalen anatomischen Position durch Anwendung aufblähbarer Manschetten ab, welche ein Aufbläh-Kissen enthalten, das aus einer einzigen Kammer besteht (Single-Kammer-Manschette). Während der Aufblähung der Single-Kammer-Manschette wird der Gasdruck in der Kammer symmetrisch in Richtung des Mittelpunktes der Extremität übertragen, was dazu führt, dass das weiche Gewebe in der Extremität vom Rand in Richtung mittig liegender Knochen zusammengepresst wird.

In der Ruhephase ist die Kompression des weichen Gewebes proportional zu der zentral orientierten Komponente des Manschetten-Drucks. Das verursacht eine allmähliche Reduktion des Lumens jedes Blutgefäßes, das an die Seite der Manschette angrenzt und somit eine allmähliche Reduktion zunächst des venösen Blutflusses und anschließend des arteriellen Blutflusses in der Extremität distal zu der Manschette.

Obwohl die Aufblähung der Single-Kammer-Manschette vorrangig eine Kompressionskraft auf das darunter liegende weiche Gewebe ausübt, werden ebenfalls Streck- und Scherkräfte in dem weichen Gewebe innen und außen vom Rand der Manschette generiert. Die Kräfte der aufgeblähten Manschette bedingen eine mechanische Reizung in dem weichen Gewebe, welche ab einem bestimmten Manschetten-Druck Schmerz und Unbehagen auslöst. Beim Ausführen einer Bewegung mit aufgeblasenem pneumatisches Aderpress-System an dem aktiven Körperglied (oft bezeichnet als blutflussbegrenzte Bewegung oder ischämische Bewegung), werden durch eine Zunahme des intramuskulären Drucks, welcher durch die Muskelkontraktion induziert wird, die Kräfte im Gewebe unter der druckbelasteten Manschette weiter verstärkt, wodurch wiederum der Schmerzreiz verstärkt und zusätzlich der Blutfluss gestört wird.

Von Nachteil ist, dass diese negativen Effekte in der Anwendung von Manschetten ein signifikantes Unbehagen darstellen, sowohl in der Ruhephase als auch während der Bewegung. Bei Bewegung hemmt der durch die Manschette hervorgerufene mechanische Schmerzreiz ebenfalls gewollte Muskelkontraktionen, wodurch die Effizienz ischämischen Trainings reduziert wird.

Es ist daher die Aufgabe der vorliegenden Erfindung ein pneumatisches Aderpress-System der eingangs genannten Art derart weiter zu entwickeln, dass es eine präzisere Reduzierung des Blutflusses in Körpergliedern bei gering anwachsendem Manschettendruck ermöglicht und somit weniger Unbehagen und Schmerzreiz sowohl in der Ruhephase als auch in der Bewegung auslöst.

Diese Aufgabe wird bei einem pneumatischen Aderpress-Systems der eingangs genannten Art dadurch gelöst, dass die mindestens zwei aufblähbaren Kammern in Längsrichtung der Manschette aufeinander folgend angeordnet sind und durch das Druck-Regelsystem unabhängig voneinander gesteuert aufblähbar sind, wobei eine erste Kammer zur Anwendung an den Muskeln und eine zweite Kammer zur Anwendung an den Blutgefäßen vorgesehen ist.

Ein solches pneumatisches Aderpress-System hat den Vorteil, dass es eine asymmetrische Kompression eines menschlichen Körperglieds ermöglicht und somit eine komfortable und exakte Steuerung des Levels der Blutfluss-Reduktion in das entsprechende Körperglied.

Das pneumatische Aderpress-System der vorliegenden Erfindung umfasst zwei wesentliche Aspekte. Der erste Aspekt stellt eine aufblähbare Manschette dar, bestehend aus mindestens zwei aufblähbaren Kammern in verschiedenen Größen und in Längsrichtung der Manschette aufeinander folgend angeordnet (Multi-Kammer-Manschette). Der zweite Aspekt stellt ein Druck-Regelsystem dar, welches die Kammern jeweils aufbläht und einen vorgegebenen Druck-Level in jeder der genannten Kammern separat steuert und dadurch unabhängige lokale Kompression des Körperteil-Gewebes unterhalb jeder der genannten Kammern ermöglicht, entsprechend dem vorgegebenen Druck in der jeweiligen Kammer. Die Manschette und das Druck-Regelsystem sind pneumatisch miteinander verbunden und bilden als eine Einheit den Gegenstand der Erfindung.

Im Gegensatz zu Standard-Single-Kammer-Manschetten presst die Multi-Kammer-Manschette das Körperglied in asymmetrischer Form zusammen, so dass verschiedene Kompressionskräfte simultan auf den gewünschten Bereich des Körperglieds angewendet werden.

Mit der vorliegenden Erfindung wurde der gegenwärtige Stand der Technik pneumatischer Aderpress-Systeme und aufblähbarer Manschetten in einer Art erweitert, welche den Bedürfnissen einer verbesserten Einschränkung des Blutflusses in menschlichen Körpergliedern, insbesondere während der Bewegung, entspricht.

Nachfolgend wird ein Ausführungsbeispiel der Erfindung anhand der Figuren näher erläutert. Es zeigen:
- Fig. 1: eine schematische Ansicht eines pneumatischen Aderpress-Systems mit den Erfindungsmerkmalen,
- Fig. 2: einen Querschnitt der Manschette gemäß Fig. 1, und
- Fig. 3: die Anwendung der Manschette gemäß Fig. 1 an den am häufigsten genutzten anatomischen Positionen am menschlichen Körper in unteren (Fig. 3A und Fig. 3B) oder oberen (Fig. 3C) Extremitäten.

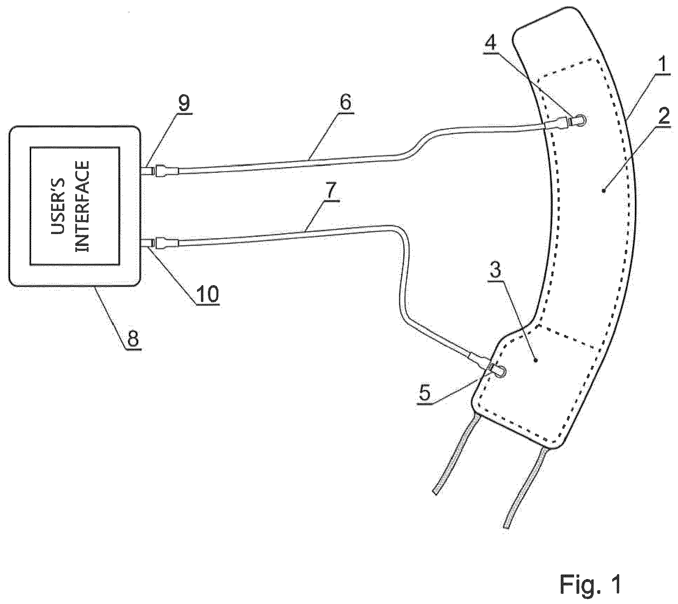

Fig. 1 zeigt eine schematische Ansicht eines pneumatischen Aderpress-Systems mit den Erfindungsmerkmalen. Das pneumatische Aderpress-System, auch als pneumatisches Tourniquet-System bezeichnet, umfasst eine Manschette 1 und ein Druck-Regelsystem 8, die pneumatisch miteinander verbunden sind.

Bei dem gezeigten Ausführungsbeispiel ist die Manschette 1 als Multi-Kammer-Manschette mit zwei aufblähbare Kammern 2, 3 ausgebildet. Die Kammern 2, 3 sind unterschiedlich groß und werden im Folgenden als große Kammer 2 und kleine Kammer 3 bezeichnet. Die große Kammer 2 und die kleine Kammer 3 sind in die Manschette 1 eingebettet und in Längsrichtung der Manschette 1 aufeinander folgend angeordnet. Die große Kammer 2 erstreckt sich über den überwiegenden Teil der Manschette 1 und ist an die langgestreckte Form der Manschette 1 angepasst. Die kleine Kammer 3 hat im nicht aufgeblähten Zustand eine im Wesentlichen quadratische Grundfläche und ist an einem Ende der Manschette 1 angeordnet. Dabei ist die große Kammer 2 zur Anwendung an den Muskeln und die kleine Kammer 3 zur Anwendung an Blutgefäßen 12 vorgesehen.

Weiterhin hat die große Kammer 2 einen ersten Manschetten-Anschluss 4 und die kleine Kammer 3 einen zweiten Manschetten-Anschluss 5.

Das Druck-Regelsystem 8 hat Anschlüsse 9, 10, zur Verbindung mit der Manschette 1. Bei dem Ausführungsbeispiel hat das Druck-Regelsystem 8 einen ersten Anschluss 9 und einen zweiten Anschluss 10.

Die Manschette 1 und das Druck-Regelsystem 8 sind durch zwei Verbindungskanäle 6, 7 pneumatisch miteinander verbunden. Dabei ist ein erster Verbindungskanal 6 an einem ersten Ende mit dem ersten Manschetten-Anschluss 4 der großen Kammer 2 verbunden und an einem von dem ersten Ende abgewandten zweiten Ende mit dem ersten Anschluss 9 des Druck-Regelsystems 8. Ein zweiter Verbindungskanal 7 ist an einem ersten Ende mit dem zweiten Manschetten-Anschluss 5 der kleinen Kammer 3 verbunden und an einem von dem ersten Ende abgewandten zweiten Ende mit dem zweiten Anschluss 10 des Druck-Regelsystems 8.

Das Druck-Regelsystem 8 kann die große Kammer 2 und die kleine Kammer 3 aufblähen und einen vorher festgelegten Druck-Level in der großen Kammer 2 und in der kleinen Kammer 3 unabhängig voneinander steuern.

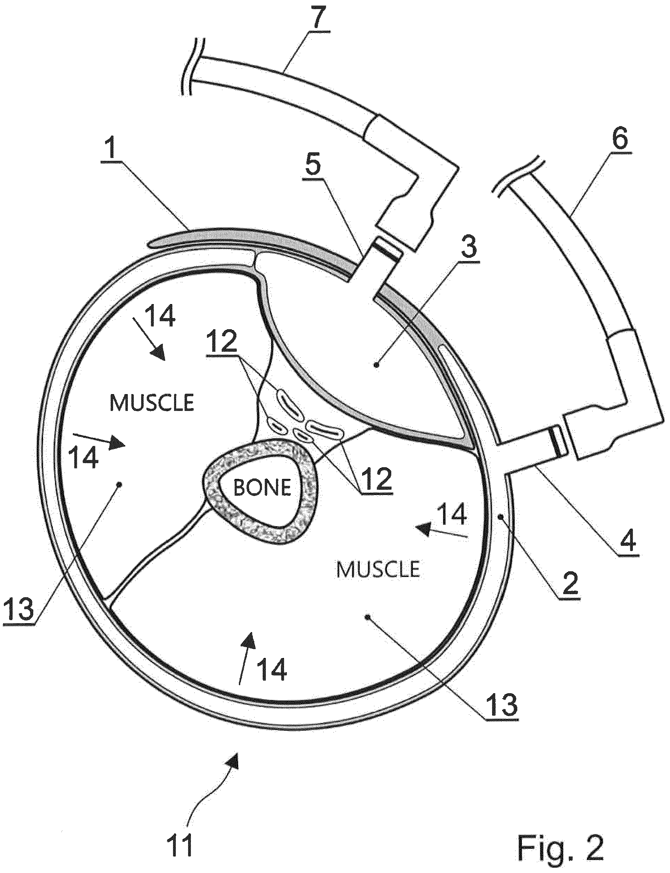

Fig. 2 zeigt einen Querschnitt einer Manschette 1 gemäß Fig. 1. Die Manschette 1 umschließt in der Abbildung ein Körperglied 11. Gemäß Fig. 1 hat die Manschette 1 die große aufblähbare Kammer 2 und die kleine aufblähbare Kammer 3, welche in Längsrichtung der Manschette 1 aufeinander folgend angeordnet sind. Die kleine Kammer 3 wird an Blutgefäßen 12 angewendet. Dabei ist die kleine Kammer 3 über dem Bereich des Körperglieds 11 angeordnet, in dem große Blutgefäße 12, die den Bereich des Körperglieds 11 distal zu der Manschette versorgen, am stärksten oberflächennah verlaufen. Die große Kammer 2 wird an Muskeln angewendet. Dabei ist die große Kammer 2 um den restlichen Umfang des Körperglieds 11 gewickelt. Der erste Verbindungskanal 6 wird mit dem ersten Manschetten-Anschluss 4 der großen Kammer 2 verbunden. Der zweite Verbindungskanal 7 wird mit dem zweiten Manschetten-Anschluss 5 der kleinen Kammer 3 verbunden.

Die kleine Kammer 3 ist gegenüber der großen Kammer 2 mit einem stärkeren Druck aufgebläht. Auf diese Weise erzeugt die kleine Kammer 3 eine größere Kompressionskraft als die große Kammer 2. Die Kompressionskraft ist von der Manschette 1 in Richtung mittig liegender Knochen gerichtet, wie in der Abbildung mit Pfeilen 14 dargestellt. Die größere Kompressionskraft, die durch die kleine Kammer 3 generiert wird, reduziert primär das Lumen der großen Blutgefäße 12. Dadurch wird der Blutfluss distal zu der Manschette 1 reduziert. Die kleinere Kompressionskraft die durch die große Kammer 2 generiert wird, wird an den Muskeln 13 des Körperglieds 11 angewendet.

Auf diese Weise kommt es zu einer asymmetrischen Kompression in dem menschlichen Körperglied 11. Bei Bewegung dämpft die geringere mechanische Kompression des Muskels 13 in dem Körperglied 11, die durch die Manschette 1 erzeugt wird, die Generation von Scherkräften im Gewebe. Dadurch wird der Schmerzreiz sowohl während der Kontraktions- als auch in der Ruhephase des Muskels 13 reduziert.

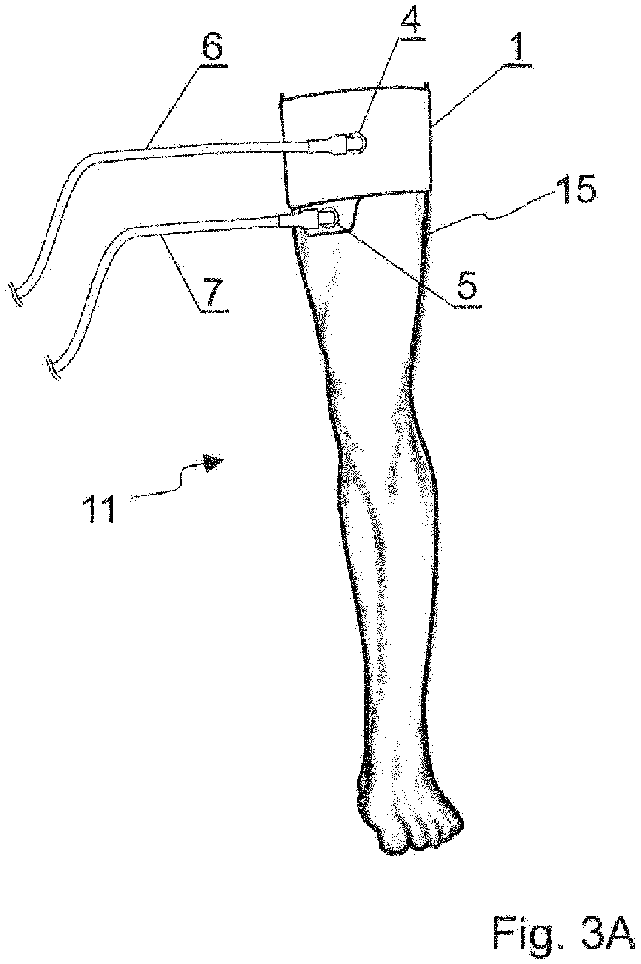

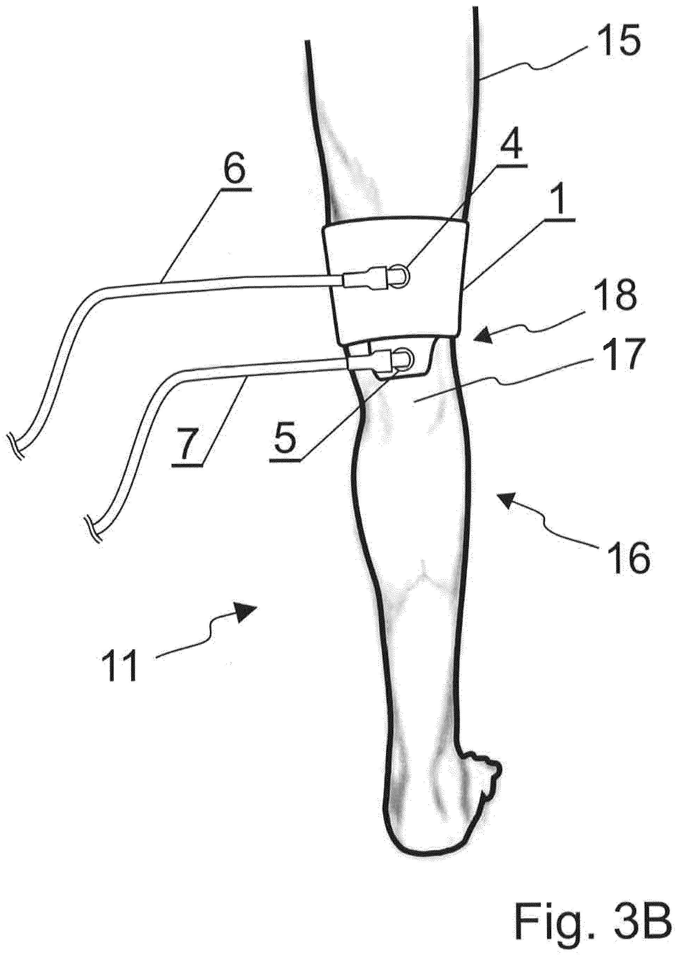

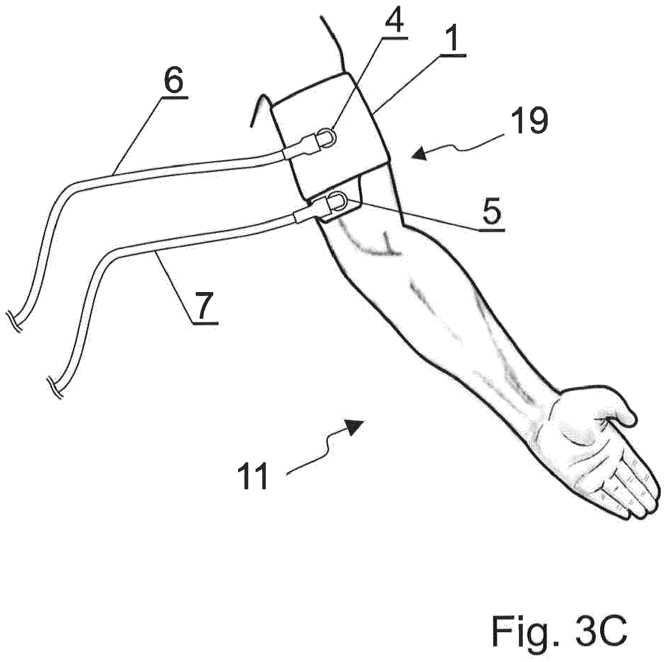

Fig. 3 zeigt die Anwendung der Manschette 1 gemäß Fig. 1 an den am häufigsten genutzten anatomischen Positionen am menschlichen Körper in unteren (Fig. 3A und Fig. 3B) oder oberen (Fig. 3C) Extremitäten. Die Manschette 1 umschließt ein Körperglied 11. Gemäß Fig. 1 hat die Manschette 1 die große aufblähbare Kammer 2 und die kleine aufblähbare Kammer 3. Der erste Verbindungskanal 6 ist mit dem ersten Manschetten-Anschluss 4 der großen Kammer 2 verbunden. Der zweite Verbindungskanal 7 ist mit dem zweiten Manschetten-Anschluss 5 der kleinen Kammer 3 verbunden.

Fig. 3A zeigt beispielhaft die Konditionierung von Oberschenkel-Muskeln. Die kleine Kammer 3 mit dem gegenüber der großen Kammer 2 höheren vorgegebenen Druck wird an einer anteromedialen Stelle eines Oberschenkels 15 positioniert. Auf diese Weise sind zentrisch orientierte Kompressionskräfte an der Arterie und der Vene des Oberschenkels 15 lokalisiert (*trigonum femorale - lacuna vasorum*). Die große Kammer 2 hat einen gegenüber der kleinen Kammer 3 geringeren vorgegebenen Druck. Die große Kammer 2 umschließt den restlichen Umfang des Oberschenkels 15. Dabei wirkt eine geringere Kompression auf die Muskel-Komponente des Oberschenkels 15.

Das gleiche Prinzip wird bei der Begrenzung des Blutflusses in Muskeln eines Unterschenkels 16 (Fig. 3B) oder oberer Extremitäten, wie beispielsweise in Muskeln eines Oberarms 19 (Fig. 3C) genutzt.

In Fig. 3B ist die kleine Kammer 3 im hinteren Bereich eines Knies 17 über den Blutgefäßen in der Region einer Kniekehle 18, im Übergang zur dem Unterschenkel 16 positioniert.

In Fig. 3C ist die kleine Kammer 3 medial (im mittleren Bereich) am Oberarm 19 über den Blutgefäßen des Oberarms 19 positioniert.

### Bezuaszeichenliste:

- 1: Manschette (Multi-Kammer Manschette)
- 2: Große Kammer
- 3: Kleine Kammer
- 4: erster Manschetten-Anschluss
- 5: zweiter Manschetten-Anschluss
- 6: erster Verbindungskanal
- 7: zweiter Verbindungskanal
- 8: Druck-Regelsystem
- 9: erster Anschluss des Druck-Regelsystems
- 10: zweiter Anschluss des Druck-Regelsystems
- 11: Körperglied
- 12: Blutgefäß
- 13: Muskel
- 14: Pfeil
- 15: Oberschenkel
- 16: Unterschenkel
- 17: Knie
- 18: Kniekehle
- 19: Oberarm

## Patentansprüche

1. Pneumatisches Aderpress-System für die Konditionierung von Muskel- und Blutgefäßen mit einer Manschette (1) mit mindestens zwei aufblähbaren Kammern (2, 3) und mit einem Druck-Regelsystem (8) zum definierten Aufblähen der Manschette (1), **dadurch gekennzeichnet, dass** die mindestens zwei aufblähbaren Kammern (2, 3) in Längsrichtung der Manschette (1) aufeinander folgend angeordnet sind und durch das Druck-Regelsystem (8) unabhängig voneinander gesteuert aufblähbar sind, wobei eine erste Kammer (2) zur Anwendung an den Muskeln und eine zweite Kammer (3) zur Anwendung an den Blutgefäßen (12) vorgesehen ist.

2. Pneumatisches Aderpress-System nach Anspruch 1, **dadurch gekennzeichnet, dass** die Kammern (2, 3) jeweils einen Manschetten-Anschluss (4, 5) haben.

3. Pneumatisches Aderpress-System nach Anspruch 1 oder 2, **dadurch gekennzeichnet, dass** das Druck-Regelsystem (8) Anschlüsse (9, 10) hat, wobei die Anzahl der Anschlüsse (9, 10) des Druck-Regelsystems (8) der Anzahl an Kammern (2, 3) der Manschette (1) entspricht.

4. Pneumatisches Aderpress-System nach einem der vorhergehenden Ansprüche, **dadurch gekennzeichnet, dass** die Manschette (1) mit Verbindungskanälen (6, 7) pneumatisch mit dem Druck-Regelsystem (8) verbunden ist und/oder die Manschette (1) zum Umschließen oder Fixieren eines oberen oder unteren Körpergliedes (11) ausgebildet ist.

5. Pneumatisches Aderpress-System nach Anspruch 4, **dadurch gekennzeichnet, dass** jeweils ein erstes Ende eines Verbindungskanals (6, 7) pneumatisch mit einem der Manschetten-Anschlüsse (4, 5) verbunden ist und ein von dem ersten Ende des jeweiligen Verbindungskanals (6, 7) abgewandtes zweites Ende des Verbindungskanals (6, 7) pneumatisch mit einem der Anschlüsse (9, 10) des Druck-Regelsystems (8) verbunden ist.

6. Pneumatisches Aderpress-System nach einem der vorhergehenden Ansprüchen, **dadurch gekennzeichnet, dass** die Kammern (2, 3) eine unterschiedliche Größe haben und als eine große Kammer (2) und eine kleine Kammer (3) ausgebildet sind, wobei vorzugsweise die große Kammer (2) und die kleine Kammer (3) in die Manschette (1) eingebettet sind.

7. Pneumatisches Aderpress-System nach Anspruch 6, **dadurch gekennzeichnet, dass**, die große Kammer (2) einen ersten Manschetten-Anschluss (4) hat und die kleine Kammer (3) einen zweiten Manschetten-Anschluss (5) hat.

8. Pneumatisches Aderpress-System nach Anspruch 7, **dadurch gekennzeichnet, dass** das Druck-Regelsystem (8) einen ersten Anschluss (9) und einen zweiten Anschluss (10) hat.

9. Pneumatisches Aderpress-System nach Anspruch 8, **dadurch gekennzeichnet, dass** der erste Manschetten-Anschluss (4) der großen Kammer (2) mit einem ersten Verbindungskanal (6) pneumatisch mit dem ersten Anschluss (9) des Druck-Regelsystems (8) verbunden ist und/oder der zweite Manschetten-Anschluss (5) der kleinen Kammer (3) mit einem zweiten Verbindungskanal (7) pneumatisch mit dem zweiten Anschluss (10) des Druck-Regelsystems (8) verbunden ist.

10. Pneumatisches Aderpress-System nach einem der vorhergehenden Ansprüche, **dadurch gekennzeichnet, dass** mittels des Druck-Regelsystems (8) der Druck innerhalb der jeweiligen Kammer (2, 3) nah an einem gegebenen ReferenzWert für jede einzelne Kammer (2, 3) und/oder nah an einem variablen DruckWert entsprechend eines vorgegebenen Programms und/oder nah an einem variablen vorgegebenen Wert angepasst an das Referenz-Feedback-Signal regulierbar ist.

11. Pneumatisches Aderpress-System nach Anspruch 10, **dadurch gekennzeichnet, dass** mittels des Druck-Regelsystems (8) die Kammern (2, 3) aufblähbar und/oder ein vorgegebener Druck-Level in der jeweiligen Kammer (2, 3) separat steuerbar und/oder eine lokal unabhängige Kompression des Gewebes in einem Körperglied (11) unter der jeweiligen Kammer (2, 3) der Manschette entsprechend dem vorher definierten Druck in der jeweiligen Kammer (2, 3) erzielbar ist.
